# EUROPEAN PATENT SPECIFICATION

(11) **EP 2 991 731 B1**
(45) Date of publication and mention of the grant of the patent: **14.11.2018**
(21) Application number: 14794429.2
(22) Date of filing: 30.04.2014
(51) Int. Cl.: A61N 5/06, H01S 3/10, H01S 3/136

(54) **MULTIPLE APERTURE HAND-HELD LASER THERAPY APPARATUS**
TRAGBARE LASERTHERAPIEVORRICHTUNG MIT MEHREREN APERTUREN
APPAREIL DE THÉRAPIE LASER PORTATIF À OUVERTURES MULTIPLES

(30) Priority: 30.04.2013 US 201313873602
(43) Date of publication of application: 09.03.2016
(73) Proprietor: Medical Coherence LLC, Fairfield, NJ 07004 (US)
(72) Inventor: GERLITZ, Yonatan, 42835 Lev Hasharon (IL)
(74) Representative: Bailey, Jennifer Ann
(86) International application number: PCT/IB2014/001638
(87) International publication number: WO 2014/181196

(56) References cited:
- US-A1- 2002 068 926
- US-A1- 2003 170 586
- US-A1- 2008 215 123
- US-A1- 2011 032 960
- US-A1- 2013 041 431
- US-A1- 2013 317 571

## Description

### BACKGROUND OF THE INVENTION

### Field of the Invention (Technical Field):

The present invention relates generally to a hand-held low energy laser apparatus for treating human and animal patients, and more particularly to such an apparatus having changeable lens mounting assemblies to permit adjustment of the effective aperture of the laser.

### Background Art:

The use of laser light to treat people and animals is known. Since the early history of mankind people have used the light from the sun to help cure ailments. In the mid-20th century attempts were made to use concentrated light to treat wounded soldiers during World War II. In later years, the laser, which is based on the quantum phenomenon of stimulated emission, provided an excellent source of concentrated coherent light for treating patients. The laser allows the therapeutic use of a selected intensity of a monochromatic and essentially coherent light beam. Such coherent light beams have been found to be effective in treating people for various ailments.

The use of a carefully selected wavelength, generally coherently directed toward a person, provides energy for selectively stimulating processes in living cells. This can help increase blood flow, excite cell activity, and intensify inter-cell communications. Laser light treatments have been applied to various ailments. Applications include various skeletal and tissue pains and injuries, such as: rheumatic and/or chronic joint inflammation; sports injuries, wounds and fresh scars; lower and upper back pain; neck pains; plantar fasciitis and sprains; tennis elbow; Achilles tendon infection; carpal tunnel syndrome; and lymphedema and edema. The treatment has also been employed in medical dermatology, such as acne, burns, scars, hemorrhoids, vitiligo (discolored skin), and herpes simplex. Lasers also find use in medical aesthetics, including the treatment of aging and dermatolysis of the face, wrinkles, sensitive skin, post-pregnancy, stretch marks, and the like. Other applications include veterinary, dental, acupuncture, and other possibilities.

The use of laser light in therapy has been shown to reduce pain, induce antiinflammatory activity, induce healing processes and induce skin rejuvenation. In the past, light therapy has been applied by large, expensive and hazardous equipment which requires operation by trained personnel. Thus miniature, user safe laser therapy devices, which can be used at home, are desirable. No known hand-held device, however, enables changes in the aperture, the treated area, and the radiant power.

Against the foregoing background, the present invention was developed. Reference is made to the following documents: US2003/0170586 A1, US2008/0215123 A1 and US2002/0068926 A1.

### SUMMARY OF THE INVENTION (DISCLOSURE OF THE INVENTION)

The invention is defined in the appended set of claims.

The present invention suggests low-cost solutions for readily changing the effective aperture and radiant power of a hand-held laser apparatus, which changes can be made by the consumer-user.

A hand-held therapeutic laser apparatus with a special opto-mechanical construction, which enables changeability of a front collimating lens to create different effective laser apertures. A smaller effective aperture has a comparatively higher radiant flux density for treatment of a small area that requires a higher energy dose, while a larger effective aperture facilitates treatment of a large area at a relatively reduced radiant intensity.

The hand-held apparatus is an eye-safety laser device, and remains eye-safe during the process of changing the aperture. In one of the embodiments, the laser apparatus uses the natural divergence of the laser diode, which enables changing the front lens length with different sizes, focal lengths and distances from the laser source. The output beam in all cases is an essentially coherent beam. According to another embodiment, a laser diode beam correction lens is attached to the laser diode, then a diverging lens is used to create a beam divergence, and then the front lens creates again an essentially coherent beam, which is changeable to a variety of apertures, focal lengths and distances from the divergence lens. This latter embodiment suggests a more complicated apparatus than the first mentioned embodiment, but offers better coherence of the output beam. Eye safety is maintained with all lenses, and of course as well as without the front lens, during the process of changing the front lens.

### BRIEF DESCRIPTION OF THE DRAWINGS

The accompanying drawings, which are incorporated into and form a part of the specification, illustrate several embodiments of the present invention and, together with the description, serve to explain the principles of the invention. The drawings are only for the purpose of illustrating preferred embodiments of the invention, and are not to be construed as limiting the invention. Further, all dimensions seen in the drawings are exemplary and not limiting of the scope of the invention. In the drawings:
Fig. 1 is a diagrammatic illustration of a hand-held low-level laser therapy apparatus according to the present disclosure, showing the emission of a collimated from the apparatus to define an illuminated area on a treatment surface; in the figure, the treatment surface is depicted in a position rotated relative to the apparatus;
Fig. 2A is a transverse sectional view of a first lens head assembly, carrying a front lens, engaged with a base assembly mounting a laser diode, in accordance with a preferred embodiment of the present invention;
Fig. 2B is a transverse sectional view of a second lens head assembly, engageable with the base assembly depicted in Fig. 2A, according to a preferred embodiment of the invention;
Fig. 2C is a transverse sectional view of a third lens head assembly, engageable with the base assembly depicted in Fig. 2A, according to a preferred embodiment of the invention;
Fig. 3A is a transverse sectional view of a first lens head assembly, carrying a front lens, engaged with a base assembly mounting a laser diode and other optics, in accordance with an alternative embodiment of the present invention;
Fig. 3B is a transverse sectional view of a second lens head assembly, engageable with the base assembly depicted in Fig. 3A, according to an alternative embodiment of the invention;
Fig. 3C is a transverse sectional view of a third lens head assembly, engageable with the base assembly depicted in Fig. 3A, according to an alternative embodiment of the invention;
Fig. 4A is a perspective schematic view of an optical system in accordance with a preferred embodiment of the invention, showing a first collimating lens located at a first position in front of a laser diode emitting a multi-directionally diverging output beam, the lens intercepting the output beam to provide an effective laser aperture with a final beam having a first set of lateral dimensions and a flux density;
Fig. 4B is a perspective schematic view of an optical system in accordance with the embodiment of FIG. 4A, showing a second collimating lens located at a second position in front of a laser diode emitting a multi-directionally diverging output beam, the lens intercepting the output beam to provide an effective laser aperture with a final beam having a second set of lateral dimensions and a second flux density;
Fig. 4C is a perspective schematic view of an optical system in accordance with the embodiment of FIG. 4A, showing a third collimating lens located at a third position in front of a laser diode emitting a multi-directionally diverging output beam, the lens intercepting the output beam to provide an effective laser aperture with a final beam having a third set of lateral dimensions and a third flux density;
Fig. 5A is a perspective schematic view of an optical system in accordance with an alternative embodiment of the invention, showing a first collimating lens located at a first position in front of an optical subsystem emitting an approximately circular diverging beam, the collimating lens intercepting the diverging beam to provide an effective laser aperture with an approximately circular final beam having a first diameter and a flux density;
Fig. 5B is a perspective schematic view of an optical system in accordance with the embodiment of FIG. 5A, showing a second collimating lens located at a second position in front of the optical subsystem of FIG. 5A emitting an approximately circular diverging beam, the second collimating lens intercepting the diverging beam to provide an effective laser aperture with an approximately circular final beam having a second diameter and a second flux density;
Fig. 5C is a perspective schematic view of an optical system in accordance with the embodiment of FIG. 5A, showing a third collimating lens located at a third position in front of the optical subsystem of FIG. 5A emitting an approximately circular diverging beam, the third collimating lens intercepting the diverging beam to provide an effective laser aperture with an approximately circular final beam having a third diameter and a third flux density; and
FIG. 6 is a partially transparent view of a hand-held low-level laser therapy apparatus according to this disclosure and similar to the embodiment of FIG. 1, showing additional features of the invention.

### DESCRIPTION OF THE PREFERRED EMBODIMENTS

### (BEST MODES FOR CARRYING OUT THE INVENTION)

There is disclosed hereby a portable therapeutic laser apparatus. The apparatus and system according to this disclosure are adapted to exploit the natural divergence of the laser beam emitted by the laser diodes known for use in small medical devices. The laser beam, such as an infrared beam, emitted by such diodes ordinarily diverges at angles in relation to the axial direction of beam transmission. For example, if the diode is "aimed" in a direction to emit a beam having a horizontal axis of beam transmission, the initially emitted beam will have a vertical angle of divergence in a vertical plane and a horizontal angle of divergence in the horizontal plane (such planes can be visualized as intersecting along the axis of beam transmission). One of the angles of divergence typically is substantially larger than the other; whether the vertical or the horizontal angle is the greater depends upon the spatial orientation of the diode. Rotation of the diode on the axis of beam transmission likewise rotates the angles of divergence.

The instant invention exploits these angles of beam divergence by using one or more lenses to subtend and intercept the diverging beam. A lens is provided that is shaped and sized to intercept both (or all) principle angles of divergence. For example, in some embodiments, an elongated toroidal lens is employed. (A toroidal, or toric, lens has a surface which forms a portion or portions of toric surfaces.) The lens is positioned, relative to the laser diode, such that the lens's major dimension is aligned with the diode's larger angle of divergence and its minor dimension is aligned with the smaller angle of divergence. The lens is sized thus to intercept at least a substantial majority if not all the divergent beam, thereby to allow the diverged beam to be collimated and directed to therapeutic application. The beam, first diverged and then collimated, is dimensionally larger with a relatively reduced intensity or radiant fluence, improving its suitability for use in therapeutics.

The hand-held therapeutic laser apparatus of this disclosure thus has a special opto-mechanical construction, which enables changeability of a front collimating lens to create different effective laser apertures. A smaller effective aperture has a comparatively higher radiant flux density for treatment of a small area that requires a higher energy dose, while a larger effective aperture facilitates treatment of a large area at a relatively reduced radiant intensity. The user is able to regulate laser dosimetry to the treated area by changing-out a lens head assembly to adjust the effective laser aperture by varying the radiant flux emitted by the apparatus.

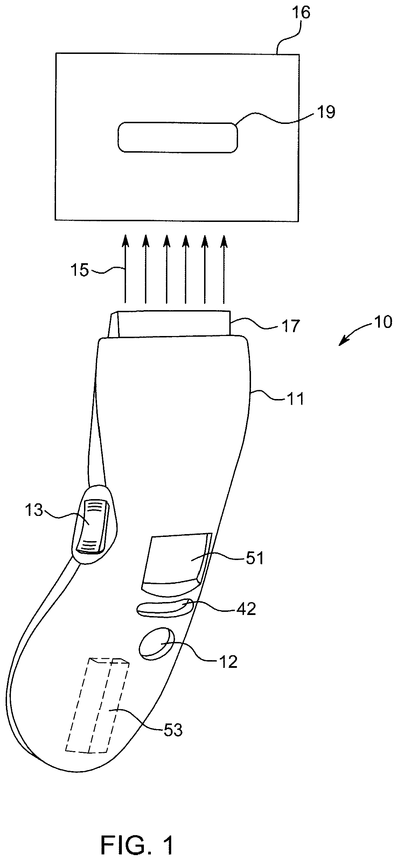

FIG. 1 is a schematic illustration of a handheld low-level laser therapy apparatus **10** for performing laser therapy, according to an exemplary embodiment of the invention. In an exemplary embodiment of the invention, apparatus **10** provides as output an elongated monochromatic substantially coherent laser beam **15** that is collimated by a lens (not shown) directly from the naturally diverging beam emitted by a laser diode within the apparatus **10.** FIG. 1 shows the laser-illuminated area **19** that is defined upon an object surface **16** (e.g., a portion of a patient's skin) by the impingement of the beam **15;** the area **19** is generally elliptical or rectangular in shape. In contrast to prior art devices, instead of focusing the laser beam from the laser diode to a single spot to provide a stronger illumination on that spot, the present system exploits the natural tendency of the laser diode to cast a diverging beam to form an elongated beam to cover a larger targeted area **19.**

For clarity of illustration in FIG. 1, the object surface **16** is rotated (e.g. 90 degrees) with respect to the directional axis of propagation of the final beam **15.** The FIG. 1 nevertheless depicts how, in one preferred embodiment, the final beam defines in a plane (i.e., **16**) perpendicular to the direction of propagation of the beam **15** an elongated illuminated area **19,** in which the length of the illuminated area is at least twice the width of the illuminated area.

A standard laser diode in use in medical devices typically has a first angle of divergence of about 5 to 10 degrees along its width, and a second angle of divergence of about 30 to about 40 degrees along its length. Instead of using a lens to correct the beam to a narrow beam, the present apparatus **10** employs a lens to form a collimated elongated beam **15** to cover a larger area **19,** for example a treated area of about 3 cm to about 6 cm by about 0.5 to 1 cm. In an exemplary embodiment of the invention, the length of the illuminated area **19** is at least twice the width of the illuminated area **19.** Further, the resulting elongated beam **15** is essentially coherent, having a light beam with an essentially common phase as accepted for laser diode emission.

By illuminating a comparatively larger area **19,** the present system illuminates each particular point in the targeted object area with a weaker and safer laser beam (for example an eye safe beam having an intensity which is not hazardous to a person's eye). More power can be delivered more accurately to a specific area by illuminating for a longer time, or increasing the intensity of the laser diode, without the user's having to move the apparatus **10** during therapy. In the contrasting use of a known single-spot type laser, a single point is illuminated intensely and an object area targeted for treatment is processed by moving the device to shift the beam across the user's skin - thus illuminating intensely (but presumably briefly and periodically) each particular point in the target area.

In an exemplary embodiment of the invention, for example as seen in FIG. 1, the light sources and electronic circuitry for powering the apparatus **10** are encased in an ergonomic encasement **11** designed to fit into a user's hand. Optionally, the apparatus **10** includes an on/off switch **12,** which turns the apparatus **10** on and off. When apparatus **10** is in the "on" state, it may be activated by pressing an activation switch **13** located on the side of encasement **11.** Alternatively or additionally, apparatus **10** may be activated by pushing on or more eye safety activation switches, located on the distal or projection end **17** of the apparatus, against the person or object being radiated while using the apparatus **10.** Activation when pressing against the person being radiated increase the safety of the apparatus, because it will not accidentally allow a user to shine light into the user's eye.

In preferred embodiments of the invention, apparatus **10** is powered by an internal power source (e.g. batteries **53**). Alternatively or additionally, the apparatus can be powered by an external power source via a power-cable (not shown) that is plugged into an external power source, such as a household power socket. Optionally, when the device is plugged into an external power source the batteries may be recharged.

In some embodiments of the invention, apparatus **10** includes a display **51,** for example an LCD display, which shows various information, such as the status of the battery, and/or a timer/counter. In an exemplary embodiment of the invention, the timer on display **51** is set by the user to a pre-selected value using a selector switch **42** of any suitable type; the value may represent an amount of time in seconds during which the apparatus will remain active when activated by the user. The apparatus may count down and deactivate automatically once it counts the pre-selected amount of time. For example if the user wishes to illuminate an object area for a specific amount of time, he sets the timer with the desired amount of time and activates the apparatus **10.** The apparatus **10** illuminates the targeted object area until the time expires.

Thus, a basic embodiment of the apparatus according to the present disclosure is a hand-held laser apparatus having a laser diode that is adapted to produce a divergent monochromatic laser beam, and a front lens adapted to receive the divergent beam directly from the laser diode and exploit the natural beam divergence of the laser diode to form an essentially coherent monochromatic, collimated output beam **15,** wherein the formed beam defines on a plane perpendicular to the direction of propagation of the beam an elongated illuminated area **19** in which the length of the illuminated area (greater dimension of area **19**) is at least twice the size of the width (shorter dimension of area **19**) of the illuminated area. The apparatus has a controller (e.g., switch **13**) that is adapted to control activation of the laser diode. The encasement **11** encloses the laser diode, the lens and the controller, and is adapted to be hand-held. The optical-mechanical configuration of the apparatus allows a changing of the front lens, and thus of the effective aperture and radiant intensity of the output beam **15,** in manners to be described further.

In all embodiments of the disclosed apparatus, there is a base assembly that mounts and includes the laser diode and, optionally, other optical components such as selected correction and/or divergent lenses. A base assembly connected in the projecting end of the apparatus hosing or encasement. All embodiments also feature at least one, preferably two or more, lens head assemblies. Each head assembly is a structural assembly mounting a front or collimating lens, which subtends a diverging beam and creates the therapeutic coherent beam that is controllably aimed at the area to be treated. The head assembly(ies) is removably attachable to the base assembly for optical cooperation with it.

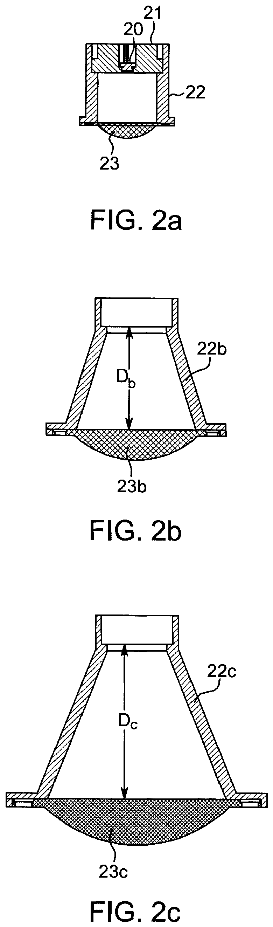

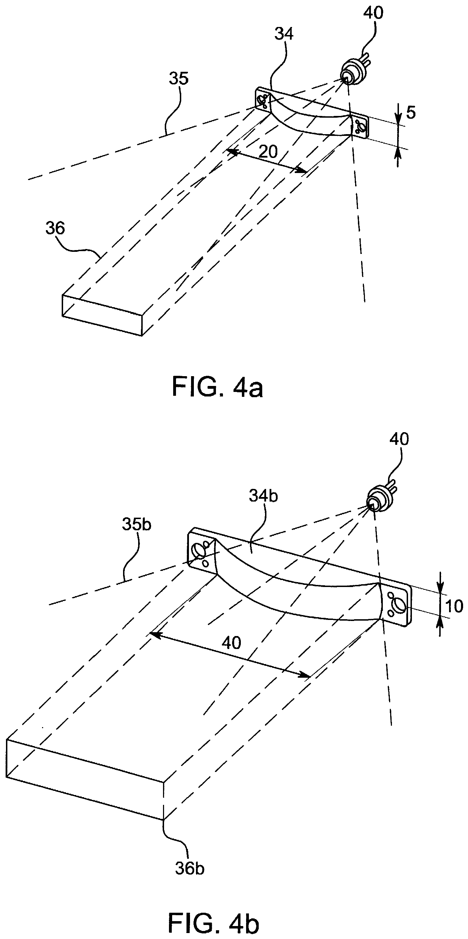

Attention is invited to FIGS. 2a-c and 4a-c, which describe the mechanical and optical arrangements in accordance with a first embodiment of the invention according to the present disclosure. FIGS. 2a-c provide information about the mechanical assemblies, while FIGS. 4a-c offer information regarding optical beam patterns. This first embodiment enables a changing of the lens and effective aperture of the hand-held laser apparatus **10.**

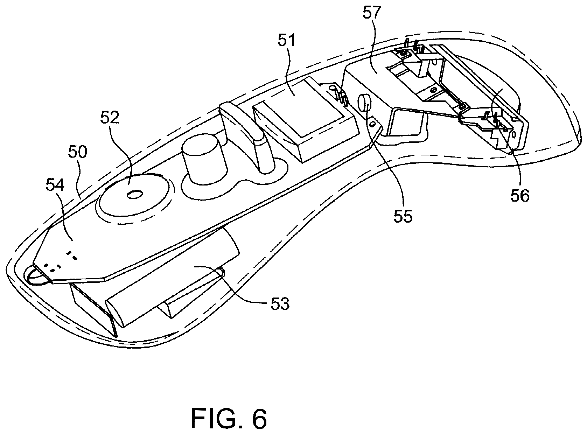

There is provided according to this disclosure mechanical assemblies attachable at, on, or near the projection end **17** of a hand-held apparatus **10** such as those seen in FIGS. 1 and 6. The assemblies are detachably provided, by any suitable attachment means such as screwed connections or clips, within or on the encasement **11** so as to controllably affect the dimensions of the emitted beam **15** and thus the effective aperture of the apparatus **10.**

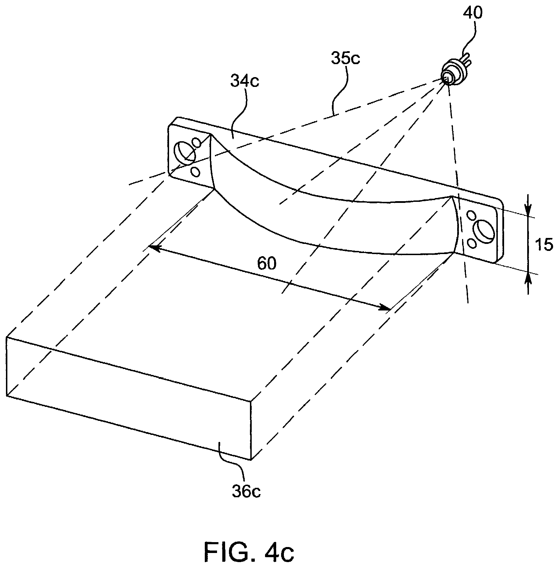

Reference is invited to FIG. 4a. The laser diode **40** has a diverging output beam **35** with a first angle of divergence of approximately 10° in one direction (i.e., in a vertical plane as depicted in FIGS. 4a-c) and a second angle of divergence of approximately 40° in the direction perpendicular to the first direction (i.e., in a horizontal plane as depicted in FIGS. 4a-c). The general boundary of the originally diverging output beam **35** is illustrated with two pairs of angled phantom lines diverging in vertical and horizontal planes from the diode **40** in FIGS. 4a-c; the first or smaller angle of divergence is in a vertical plane, while the larger, second angle of divergence is in the horizontal plane, the principle dimension of the lens **34, 34b, 34c** also being arranged at the horizontal. The divergence creates an approximately elliptically shaped beam. Lens **34** preferably is a toroidal lens which corrects the laser astigmatism, and as seen in FIG. 4 has a generally rectangular shape (in a cross section orthogonal to the axis of beam propagation). FIGS. 4a-c indicate how in one embodiment of the invention the toroidal lens **34, 34b,** or **34c** is oriented relative to the diode **40** so that the lens's minor dimension is generally aligned with the first (smaller) angle of divergence, and the lens's major dimension is generally aligned with the second (larger) angle of divergence. The lens **34** intercepts and modifies the originally emitted diverging beam **35** to create a generally rectangular final beam **36.** The size of the modified final beam **36** can be controllably changed by moving the lens **34** further from or closer to the laser diode **40.** The lens **34** is designed, according to known optical principles, with a size such that in all its operating positions on an apparatus **10** it intercepts or captures the diverging original beam **35.**

FIGS. 4a, 4b, and 4c show examples of such a design, with progressively larger lenses **34, 34b, 34c,** and with their respective created collimated final beams **(36, 36b, 36c)** manifesting lateral dimensions of, for example, first vertical dimensions of for example 5, 10, and 15, (e.g. millimeters) and second or horizontal dimensions of for example 20, 40, 60. As suggested by the figures, a larger lens (e.g. **34b** or **34c**) typically is disposed in the system correspondingly farther (in FIGS. 4b-c, respectively) from the laser diode **40** along the principal axis of beam transmission. The effective aperture of an apparatus **10,** as characterized by the dimensions (orthogonal to the principal axis of beam transmission) of the collimated final beam **36, 36b,** or **36c** created by the collimating lenses accordingly can be regulated or adjusted. By switching out on a base assembly different interchangeable head assemblies, a first final beam may be generated having a lateral beam dimension different from a corresponding lateral dimension of a second final beam created by another head assembly. A lateral beam dimension is a beam dimension (e.g. vertical or horizontal as suggested in FIG. 4a-c) measured in a plane perpendicular to the axis of principal beam transmission.

FIG. 2a shows the mechanical aperture-changing assembly for an embodiment of a multi-aperture laser apparatus according to this disclosure, including details regarding the configuration of the assembly relating with the optics of FIG. 4. In a mechanical aperture-changing assembly, the laser diode **20** is mounted on a base **21** which serves as a mechanical reference or base for the interchangeable front head lens mount **22,** which in turn includes the front lens **23** for the assembly. The front lenses **23, 23b,** and **23c** seen in FIGS. 2a-c respectively, preferably are toriodal lenses shaped generally as shown. The base **21** is situated appropriately in the apparatus **10.** Referring to FIG. 6, for example, the base **21** of FIG. 2a may correspond functionally to the base **57,** and the lens **23** of FIG. 2a may correspond to the lens **56** seen in FIG. 6. In the illustrated embodiment, the base **21** also functions as a beneficial heat sink. The laser diode **20** (corresponding generally to diode **40** in FIGS. 4a-c and diode **55** in FIG. 6) is provided on the base **21.** The front lens **23** (corresponding to lens **34** in FIG. 4a) is disposed on the changeable lens mount **22.**

Combined reference to FIGS. 2a-c provides examples of how an interchangeable changeable front head assembly, including the lens mount **22** and front lens **23,** permit the effective aperture of a hand held laser apparatus (i.e., **10, 50**) to be adjusted by controllably changing the size of the collimating lens and its distance from the laser source **20.** In FIG. 2a, the front lens **23** of a selected optical characteristic has first selected dimensions, and is carried by the lens mount **22.** The lens mount **22** is configured to as to dispose the front lens **23** a predetermined distance from the laser source **20** when the changeable front head assembly is engaged upon the base **21.** The lens mount **22** is adapted to receive and hold the particular front lens **23** having the desired optical characteristics, including lens dimensions. The lens mount **22** is removably engageable (as by, for example only, a clip or threaded screwed connection) with the heat sink base **21** mounting the laser diode **20.** When it is desired to change the size and/or intensity of the final beam (e.g., beam **36** in FIG. 4a, corresponding to beam **15** in FIG. 1), the first front head assembly is removed from the base **21,** and replaced with an interchangeable second front head assembly, such as that seen in FIG. 2b. The second front head assembly has a lens mount **22b** that is removably engageable with the base **21** in the same manner as the first lens mount **22.** The second lens mount **22b** has a greater axial dimension, however, in a manner so as to locate the front lens **23b** a relatively greater (predetermined) distance **Db** from the laser diode **20** (FIG. 2b). Further, as indicated in FIG. 2b, the second lens mount **22b** typically mounts a second front lens **23b** having optical characteristics (for example, principal dimensions) distinct from the characteristics of the first front lens **23** of FIG. 2a. FIG. 2c provides additional information regarding the advantages of the invention. The third front head assembly seen in FIG. 2c also is removably connectable to the base assembly **21.** For example, combined reference to FIGS. 2a-c shows that the first, second, and third front head assemblies all have base barrel portions of substantially equal inside diameters, which inside diameters are slightly larger than the outside diameter of the base assembly **21.** Thus the front head assemblies are essentially interchangeable with respect to the base assembly **21,** such that any one of them is disposable around, to be removably engageable with, the base **21.** FIG. 2c shows that the third lens mount **22c** positions a third front lens **23c,** of a comparatively even larger size, a relatively greater distance **Dc** from the laser diode **20,** which remains in an unchanged location and position in the base **21.**

It is understood that any suitable means for detachably connecting a head assembly including a lens mount **(22, 22b, 22c)** to a base assembly may be utilized in the practice of the invention.

From the foregoing, it is apparent that the effective aperture of a hand held therapeutic laser apparatus can be selectively changed, so as to controllably adjust the actual size (and radiant flux) of the final collimated beam **36** (i.e., beam **15** in FIG. 1). The lens **23** can be changed out to be larger, and further from the laser beam source, due to the divergent character of beam as originally emitted from the diode. The size of the area **19** illuminated on the object surface **16** thus may be regulated by the user by deploying two or more interchangeable front head assemblies.

Thus, and as more fully disclosed in co-pending senior U.S. Patent Application Serial No. 12/534,878, there is provided in the present apparatus system a laser diode that is adapted to produce a monochromatic laser beam, which may be an infrared laser. At least one lens in the system is adapted to receive the original beam directly from the laser diode, and is sized, located and shaped to exploit the natural divergence of the beam emitted from the diode to form a substantially coherent monochromatic, collimated beam of reduced flux density. The beam (e.g. beam **15**) formed by the at least one lens is adapted to form, on a plane (e.g. surface **16**) generally perpendicular to the direction of beam propagation an elongated illuminated area (e.g. area **19**). The length of the elongated illuminated (elliptical/rectangular) area preferably is at least twice the size of the width of the illuminated area.

An encasement mostly encloses the laser diode, the front collimating lens and a controller or switching adapted to control activation of the laser diode. The encasement is adapted to be hand held by the user. The lens may be a toroidal lens having differing or varying lens radii, in the direction producing the length of the illuminated area and in the direction producing the width of the illuminated area respectively. An aspect of the optical system of the invention is that the front lens is sized, shaped, and located so as to receive intercept a substantial majority or all a divergent emitted beam, so as to collimate the divergent beam and direct it to the area **19** of interest.

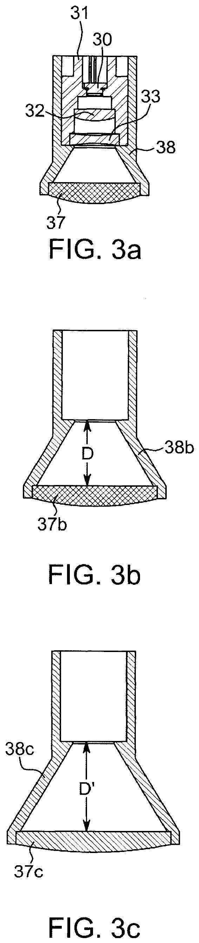

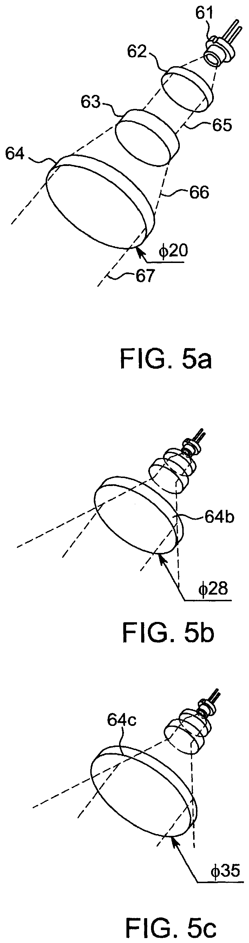

Reference is made to FIGS. 3 and 5, which are respectively the mechanical and the optical configurations of a possible second embodiment of a system according to this disclosure. In this embodiment, a laser correction lens is employed to collimate the diverging beam originally emitted from the laser diode, and to direct the resulting coherent beam to a divergent lens. The beam propagating from the divergent lens has relatively reduced radiant flux, and is then intercepted by a front collimating lens, for coherent transmission to the targeted surface. The laser diode, laser correction lens, and divergent lens preferably are arranged in a base, and the front collimating lens is disposed in a head assembly removably attachable to the base. In this alternative embodiment, the lenses may be configured so to generate a generally circular, rather than elliptical-rectangular final, substantially coherent, beam.

Regarding the optics of this alternative embodiment, the laser diode **61** in FIG. 5a emits a beam with a dimensional ratio of, for example, about 1:4 in the first (parallel) and second (perpendicular) directions. Lens **62** is a laser correction lens which corrects the astigmatism of the laser diode and its generated original beam; the corrective lens emits an approximately parallel first coherent beam **65.** A suitable such laser correction lens **62** is commercially available from CVI Melles Griot, Albuquerque, NM 87123, USA. Lens **63,** in front of the correction lens, is a divergent lens which preferably creates an approximately circular second diverging beam **66.** The front collimating lens **64** in FIG. 5a modifies the second diverging beam **66,** and creates an approximately coherent final beam **67.** As suggested by FIGS. 5a-c, the front collimating lens **64** can be provided in different sizes, and positioned at different distances from the divergent lens **63.** The size of the collimating lens **64** in each deployment distance is designed to capture substantially all the second diverging beam **66** emitted from the divergent lens **63.** Such a configuration, whereby most or all the second diverging beam **66** is intercepted and collimated, enables the collimating lens **64** to be changed (and its position changed) in a deliberate manner to create a smaller or larger effective aperture (i.e., corresponding to the diameter of the finally created mostly coherent beam **67**), according to the desired treatment area (e.g., illuminated area **19** in FIG. 1).

FIG. 3 supplies details regarding the mechanical configuration of the system relating with the optics of FIGS. 5a-c. The laser diode **30** (corresponding generally to diode **61** in FIG. 5a), the correction lens **32** (corresponding generally to correction lens **62** in FIG. 5a) and divergent lens **33** (e.g., corresponding to lens **63** in FIG. 5a) are assembled together in mechanical base assembly **31.** The base assembly may be within, for example, an encasement **11** similar to that of FIG. 1, near the projecting end **17** of the apparatus **10.** The head assembly includes a front collimating lens **37** (e.g. lens **64** in FIG. 5a, corresponding functionally to a lens in, for example, an apparatus **10** similar to that of FIG. 1) disposed on a changeable lens mount **38.**

Combined reference to FIGS. 3a-c provide examples of how an interchangeable front head assembly including a lens mount **38** and front collimating lens **37** permit adjustability in the size of the collimating lens and its distance from the divergent lens **33.** In FIG. 3a, the front collimating lens **37** has a first selected diameter, and is carried by the lens mount **38.** The lens mount **38** is adapted to receive and hold the particular collimating lens **37** having the desired optical characteristics, including, e.g., diameter. The lens mount **38** is removably engageable (as by, for example only, a clip or friction connection, or by threaded screwed engagement) with the mechanical base assembly **31** containing other optical elements of the apparatus. When it is desired to change the size and/or intensity of the final substantially coherent beam (e.g., beam **67** in FIG. 5a), the first head assembly is removed from the base assembly **31,** and replaced with an interchangeable second front head, such as that seen in FIG. 3b. The second front head has a lens mount **38b** that is removably engageable with the mechanical assembly in the same manner as the first lens mount **38.** The second lens mount **38b** has, in the example of FIG. 3b, a greater axial dimension, however, in a manner so as to locate the collimating lens **37b** a relatively greater distance **D** from the divergent lens **33** (FIG. 3b) (and thus from the source laser diode **30**). Further, as indicated in FIG. 3b, the second lens mount **38b** mounts a second collimating lens **37b** that ordinarily has different optical characteristics (for example, diameter) distinct from the characteristics of the first front collimating lens **37** of FIG. 3a. FIG. 3c provides additional illustration in this regard. The third front head assembly seen in FIG. 3c also is removably connectable to the base assembly **31.** For example, combined reference to FIGS. 3a-c shows that the first, second, and third head assemblies all respectively have base barrel portions of substantially equal inside diameters, which inside diameters are slightly larger than the outside diameter of the base assembly **31.** Thus the heads assemblies are essentially interchangeable with respect to the base assembly **31,** such that any one of them is disposable around, to be removably engageable with, the base assembly. FIG. 3c shows that the third lens mount **38c** positions a third collimating lens **37c,** having (for example) a comparatively even larger diameter, a relatively greater third distance **D'** from the divergent lens **33** (which remains in an unchanged location and position in relation with the base assembly **31.**

It is observed, therefore, that by removing and replacing on a base assembly different interchangeable head assemblies, a first final beam **67** may be generated having a lateral beam dimension different from a corresponding lateral dimension of a second final beam created by another head assembly. FIGS. 5a-c illustrate by way of example adjusted final beam diameters of 20mm, 28mm, and 35mm, generated by respective collimating lenses of correspondingly larger respective sizes and distances from the base assembly.

It is understood that any suitable means for detachably connecting a head assembly including a lens mount **(38, 38b, 38c)** to a base assembly may be utilized in the practice of the invention.

Thus, the effective aperture of a hand held therapeutic laser apparatus can be selectively changed, so as to controllably adjust the actual size (and radiant energy fluence) of the final collimated beam **67** (i.e., beam **15** in FIG. 1) created by the collimating lens. The front collimating lens **37** can be changed out to be larger, and further from the laser beam source, due to the character of the diverging beam **66** received from the divergent lens **63.** The size of the area **19** illuminated on the object surface **16** thus may be regulated by the user by deploying two or more interchangeable front head assemblies.

FIG. 6 shows generally a complete hand-held laser apparatus **50** generally in accordance with the embodiments described above. The laser apparatus **50** incorporates within its interior a printed electronic circuit board **54,** with the required electronics (known in the art) to drive the laser diode and control the operation of the laser apparatus. The lens **56** (e.g., corresponding to lens **34** in FIGS. 4a-c) is positioned in front of the laser diode **55,** which is mounted on heat sink base **57.**

The laser apparatus **50** preferably includes thereon a readily visible LCD display **51** which shows the apparatus cumulative or timed operating time, and user warnings regarding the statuses of the laser and battery **53.** The apparatus **50** preferably is powered by internally housed rechargeable batteries **53.** The apparatus **50** also optionally includes audio warnings or indicators indications available by a speaker or buzzer **52.**

In summary of the foregoing, there is described a therapeutic laser apparatus **10, 50** having an encasement **11** adapted to be hand-held. There is base assembly in the encasement, which base assembly includes a laser diode (**20, 30, 40, 55** or **61**) for generating a monochromatic laser beam whereby a diverging laser beam is emitted from the base assembly.

At least two head assemblies are removably enagageable with the base assembly, and each head assembly features a lens mount (**22, 22b, 22c, 38, 38b,** or **38c**) having an axial dimension and a collimating lens (**23, 23b, 23c, 34, 34b, 34c, 37, 37b, 37c, 64, 64b,** or **64c**) on the lens mount for creating an approximately coherent final beam. In a preferred embedment, the axial dimension of any particular lens mount of a first one of the head assemblies is different from the axial dimension of the lens mount of any given second one of the head assemblies; similarly, the size of the collimating lens of that first head assembly is different from the size of the collimating lens of the second head assemblies. As a result, when the first one of the head assemblies is engaged with the base assembly, the collimating lens of the first head assembly intercepts, at a first distance (e.g. distance **D_{b}** in FIG. 2b, or distance **D** in FIG. 3b), the diverging beam emitted from the base assembly. When the second head assembly is engaged with the base assembly, the collimating lens of the second head assembly intercepts, at a second distance (e.g. distance **D_{c}** in FIG. 2c, or distance **D'** in FIG. 3c), the diverging beam emitted from the base assembly. Consequently, the first head assembly when in use with the base assembly creates a first final beam (e.g., beam **36b** or beam **67b**) having a lateral beam dimension different from a corresponding lateral dimension of a second final beam (e.g., beam **36c** or beam **67c**) created by the second head assembly when in use with the base assembly.

The head assemblies preferably are a plurality of head assemblies that are interchangeable in relation to a particular base assembly. Any one of the head assemblies when in use with the base assembly preferably emits a coherent final beam having a radiant flux different from the radiant flux emitted from any selected other one of the head assemblies when in use with that base assembly.

In an alternative embodiment of the apparatus, the base assembly includes a correction lens **(32, 62)** for correcting astigmatism in the diverging original beam emitted from the laser diode **(40, 61)** and for modifying the original beam to create a substantially circular coherent beam **65.** In front of the correction lens is disposed a divergent lens **(33, 63)** for creating, from the generally coherent beam **65** created by the correction lens, the diverging laser beam **66** emitted from the base assembly, so that the diverging laser beam emitted from the base assembly is an approximately circular diverging beam.

In all embodiments, any one of the head assemblies when in use with a base assembly emits a coherent final beam having a radiant flux different from the radiant flux emitted from any other one of the head assemblies when in use with the base assembly. This is because when a first head assembly is used with the base assembly, it creates a first final beam having a lateral beam dimension different from a corresponding lateral dimension of any other, second, final beam created by another, different head assembly when in use with the base assembly.

Although the invention has been described in detail with particular reference to these preferred embodiments, other embodiments can achieve the same results. From the foregoing, those skilled in the art will recognize an advancement of this invention in the relevant field of art. While the invention has been described in relation to a preferred embodiment thereof shown in the accompanying drawings, it also is to be recognized that the same is readily susceptible to modification, variation and substitution of equivalents without avoiding the invention. The system and apparatus are not intended to be limited by the foregoing except as may appear in the following appended claims, and it is intended to cover in the claims all such modifications and equivalents.

## Claims

1. A therapeutic laser apparatus comprising:
an encasement (11) adapted to be hand-held;
a base assembly (21, 31) in the encasement, the base assembly comprising a laser diode (20, 30, 40, 61) configured to generate a monochromatic laser beam and the base assembly being configured to emit a diverging laser beam; and
at least two head assemblies removably enagageable with the base assembly, each head assembly comprising:
a lens mount (22, 38) having an axial dimension (D); and
a collimating lens (23, 37, 64) on the lens mount configured to create an approximately coherent, collimated final beam;
the axial dimension of the lens mount of a first one of the head assemblies being different from the axial dimension of the lens mount of a second one of the head assemblies, and a size of the collimating lens of the first one of the head assemblies being different from a size of the collimating lens of the second one of the head assemblies;
when the first one of the at least two head assemblies is engaged with the base assembly, the collimating lens of the first head assembly intercepting, at a first distance from the base assembly, the diverging beam emitted from the base assembly, and when the second one of the at least two head assemblies is engaged with the base assembly, the collimating lens of the second head assembly intercepting, at a second distance from the base assembly different from the first distance, the diverging beam emitted from the base assembly;
when in use with the base assembly, the first head assembly creating a first collimated final beam having a lateral beam dimension different from a corresponding lateral beam dimension of a second collimated final beam created by the second head assembly when in use with the base assembly.

2. The apparatus according to claim 1 wherein:
the at least two head assemblies comprise a plurality of head assemblies interchangeable in relation to the base assembly; and
any one of the head assemblies when in use with the base assembly emits a substantially coherent, collimated final beam having a radiant flux different from a radiant flux emitted from any other of the head assemblies when in use with the base assembly.

3. The apparatus according to claim 1 wherein:
the diverging laser beam emitted from the base assembly is emitted directly from the laser diode, and propagates with a first angle of divergence and with a second angle of divergence greater than the first angle of divergence; and
the collimating lens is an oblong toroidal lens having a major dimension aligned with the second angle of divergence.

4. The apparatus according to claim 1 wherein the final beam forms on a plane perpendicular to the direction of propagation of the beam an elongated illuminated area in which the length of the illuminated area is at least twice the width of the illuminated area.

5. The apparatus according to claim 1 wherein the base assembly further comprises:
a correction lens (32, 62) configured to correct astigmatism in a diverging original beam emitted from the laser diode and to modify the original beam to create an approximately circular coherent beam; and
a divergent lens (33, 63) configured to create, from the coherent beam created by the correction lens, the diverging laser beam emitted from the base assembly;
the diverging laser beam emitted from the base assembly being an approximately circular diverging beam.

6. The apparatus according to claim 5 wherein the collimating lens on the lens mount emits an approximately circular final beam.

7. The apparatus according to claim 1 having an adjustable effective laser aperture, the base assembly further comprising:
a correction lens (32, 62) configured to modify a diverging original beam emitted from the laser diode to correct beam astigmatism and to create an approximately circular, coherent, collimated beam; and
a divergent lens (33, 63) configured to create, from the coherent beam created by the correction lens, the diverging laser beam emitted from the base assembly; the diverging laser beam emitted from the base assembly being approximately circular; the head assemblies being interchangeable in relation to the base assembly;
when the first one of the at least two head assemblies is engaged with the base assembly, the collimating lens of the first head assembly intercepting, at the first distance from the base assembly, substantially all the diverging beam emitted from the base assembly, and when the second one of the at least two head assemblies is engaged with the base assembly, the collimating lens of the second head assembly intercepting, at the second distance from the base assembly different from the first distance, substantially all the diverging beam emitted from the base assembly;
when in use with the base assembly, any one of the head assemblies emitting a substantially coherent, collimated final beam having a radiant flux different from a radiant flux emitted from any other of the head assemblies when in use with the base assembly.

8. An apparatus according to claim 1, the collimating lens on the lens mount being configured to create a substantially coherent, collimated final beam, the final beam defining on a plane perpendicular to the direction of propagation of the beam an elongated illuminated area in which the length of the illuminated area is at least twice the size of the width of the illuminated area.

9. The apparatus according to claim 8 wherein:
the at least two head assemblies comprise a plurality of head assemblies interchangeable in relation to the base assembly; and
any one of the head assemblies when in use with the base assembly is configured to emit a substantially coherent final beam having a radiant flux different from a radiant flux emitted from any other of the head assemblies when in use with the base assembly.

10. The apparatus according to claim 8 wherein:
the diverging laser beam emitted from the base assembly is emitted directly from the laser diode, and propagates with a first angle of divergence and with a second angle of divergence greater than the first angle of divergence; and
the collimating lens is an oblong toroidal lens having a major dimension aligned with the second angle of divergence.

## Patentansprüche

1. Therapeutische Laservorrichtung, die Folgendes umfasst:
ein Gehäuse (1), das dafür eingerichtet ist, in der Hand gehalten zu werden,
eine Grundbaugruppe (21, 31) in dem Gehäuse, wobei die Grundbaugruppe eine Laserdiode (20, 30, 40, 61) umfasst, die dafür konfiguriert ist, einen monochromatischen Laserstrahl zu erzeugen, und die Grundbaugruppe dafür konfiguriert ist, einen divergierenden Laserstrahl abzustrahlen, und
wenigstens zwei Kopfbaugruppen, die abnehmbar mit der Grundbaugruppe in Eingriff gebracht werden können, wobei jede Kopfbaugruppe Folgendes umfasst:
eine Linsenfassung (22, 38), die eine axiale Abmessung (D) aufweist, und
eine Kollimatorlinse (23, 37, 64) an der Linsenfassung, die dafür konfiguriert ist, einen annähernd kohärenten, gebündelten Endstrahl zu erzeugen,
wobei sich die axiale Abmessung der Linsenfassung einer ersten der Kopfbaugruppen von der axialen Abmessung der Linsenfassung einer zweiten der Kopfbaugruppen unterscheidet und sich eine Größe der Kollimatorlinse der ersten der Kopfbaugruppen von einer Größe der Kollimatorlinse der zweiten der Kopfbaugruppen unterscheidet,
wenn die erste der wenigstens zwei Kopfbaugruppen mit der Grundbaugruppe in Eingriff gebracht ist, die Kollimatorlinse der ersten Kopfbaugruppe, bei einer ersten Entfernung von der Grundbaugruppe, den divergierenden Strahl, der von der Grundbaugruppe abgestrahlt wird, abfängt, und wenn die zweite der wenigstens zwei Kopfbaugruppen mit der Grundbaugruppe in Eingriff gebracht ist, die Kollimatorlinse der zweiten Kopfbaugruppe, bei einer zweiten Entfernung von der Grundbaugruppe, die sich von der ersten Entfernung unterscheidet, den divergierenden Strahl, der von der Grundbaugruppe abgestrahlt wird, abfängt,
wenn sie sich mit der Grundbaugruppe in Verwendung befindet, die erste Kopfbaugruppe einen ersten gebündelten Endstrahl erzeugt, der eine seitliche Strahlabmessung aufweist, die sich von einer entsprechenden seitlichen Strahlabmessung eines zweiten gebündelten Endstrahls unterscheidet, der durch die zweite Kopfbaugruppe erzeugt wird, wenn sie sich mit der Grundbaugruppe in Verwendung befindet.

2. Laservorrichtung nach Anspruch 1, wobei:
die wenigstens zwei Kopfbaugruppen eine Vielzahl von Kopfbaugruppen umfassen, die im Verhältnis zu der Grundbaugruppe austauschbar sind, und
eine beliebige der Kopfbaugruppen, wenn sie sich mit der Grundbaugruppe in Verwendung befindet, einen im Wesentlichen kohärenten, gebündelten Endstrahl erzeugt, der einen Strahlungsfluss aufweist, der sich von einem Strahlungsfluss unterscheidet, der von einer beliebigen anderen der Kopfbaugruppen abgestrahlt wird, wenn sie sich mit der Grundbaugruppe in Verwendung befindet.

3. Laservorrichtung nach Anspruch 1, wobei:
der divergierende Laserstrahl, der von der Grundbaugruppe abgestrahlt wird, unmittelbar von der Laserdiode abgestrahlt wird und sich mit einem ersten Divergenzwinkel und mit einem zweiten Divergenzwinkel, der größer ist als der erste Divergenzwinkel, ausbreitet und
die Kollimatorlinse eine längliche torische Linse ist, die eine größere Ausdehnung aufweist, die mit dem zweiten Divergenzwinkel ausgerichtet ist.

4. Laservorrichtung nach Anspruch 1, wobei der Endstrahl auf einer Ebene, senkrecht zu der Ausbreitungsrichtung des Strahls, einen länglichen beleuchteten Bereich bildet, in dem die Länge des beleuchteten Bereichs wenigstens das Doppelte der Breite des beleuchteten Bereichs beträgt.

5. Laservorrichtung nach Anspruch 1, wobei die Grundbaugruppe ferner Folgendes umfasst:
eine Korrekturlinse (32, 62), die dafür konfiguriert ist, einen Astigmatismus in einem divergierenden ursprünglichen Strahl, der von der Laserdiode abgestrahlt wird, zu korrigieren und den ursprünglichen Strahl zu modifizieren, um einen annähernd kreisförmigen kohärenten Strahl zu erzeugen, und
eine Zerstreuungslinse (33, 63), die dafür konfiguriert ist, aus dem durch die Korrekturlinse erzeugten kohärenten Strahl den divergierenden Laserstrahl zu erzeugen, der von der Grundbaugruppe abgestrahlt wird,
wobei der divergierende Laserstrahl, der von der Grundbaugruppe abgestrahlt wird, ein annähernd kreisförmiger divergierender Strahl ist.

6. Laservorrichtung nach Anspruch 5, wobei die Kollimatorlinse an der Linsenfassung einen annähernd kreisförmigen Endstrahl abstrahlt.

7. Laservorrichtung nach Anspruch 1, die eine einstellbare wirksame Laseröffnung aufweist, wobei die Grundbaugruppe ferner Folgendes umfasst:
eine Korrekturlinse (32, 62), die dafür konfiguriert ist, einem divergierenden ursprünglichen Strahl, der von der Laserdiode abgestrahlt wird, zu modifizieren, um einen Strahlastigmatismus zu korrigieren und um einen annähernd kreisförmigen, kohärenten, gebündelten Strahl zu erzeugen, und
eine Zerstreuungslinse (33, 63), die dafür konfiguriert ist, aus dem durch die Korrekturlinse erzeugten kohärenten Strahl den divergierenden Laserstrahl zu erzeugen, der von der Grundbaugruppe abgestrahlt wird, wobei der divergierende Laserstrahl, der von der Grundbaugruppe abgestrahlt wird, annähernd kreisförmig ist,
wobei die Kopfbaugruppen im Verhältnis zu der Grundbaugruppe austauschbar sind,
wenn die erste der wenigstens zwei Kopfbaugruppen mit der Grundbaugruppe in Eingriff gebracht ist, die Kollimatorlinse der ersten Kopfbaugruppe, bei der ersten Entfernung von der Grundbaugruppe, im Wesentlichen den gesamten divergierenden Strahl, der von der Grundbaugruppe abgestrahlt wird, abfängt, und wenn die zweite der wenigstens zwei Kopfbaugruppen mit der Grundbaugruppe in Eingriff gebracht ist, die Kollimatorlinse der zweiten Kopfbaugruppe, bei der zweiten Entfernung von der Grundbaugruppe, die sich von der ersten Entfernung unterscheidet, im Wesentlichen den gesamten divergierenden Strahl, der von der Grundbaugruppe abgestrahlt wird, abfängt,
wenn sie sich mit der Grundbaugruppe in Verwendung befindet, eine beliebige der Kopfbaugruppen einen im Wesentlich kohärenten, gebündelten Endstrahl abstrahlt, der einen Strahlungsfluss aufweist, der sich von einem Strahlungsfluss unterscheidet, der von einer beliebigen anderen Kopfbaugruppe abgestrahlt wird, wenn sie sich mit der Grundbaugruppe in Verwendung befindet.

8. Laservorrichtung nach Anspruch 1,
wobei die Kollimatorlinse an der Linsenfassung dafür konfiguriert ist, einen im Wesentlichen kohärenten, gebündelten Endstrahl zu erzeugen, wobei der Endstrahl auf einer Ebene, senkrecht zu der Ausbreitungsrichtung des Strahls, einen länglichen beleuchteten Bereich definiert, in dem die Länge des beleuchteten Bereichs wenigstens das Doppelte der Breite des beleuchteten Bereichs beträgt.

9. Laservorrichtung nach Anspruch 8, wobei:
die wenigstens zwei Kopfbaugruppen eine Vielzahl von Kopfbaugruppen umfassen, die im Verhältnis zu der Grundbaugruppe austauschbar sind, und
eine beliebige der Kopfbaugruppen, wenn sie sich mit der Grundbaugruppe in Verwendung befindet, dafür konfiguriert ist, einen im Wesentlichen kohärenten, gebündelten Endstrahl zu erzeugen, der einen Strahlungsfluss aufweist, der sich von einem Strahlungsfluss unterscheidet, der von einer beliebigen anderen der Kopfbaugruppen abgestrahlt wird, wenn sie sich mit der Grundbaugruppe in Verwendung befindet.

10. Laservorrichtung nach Anspruch 8, wobei:
der divergierende Laserstrahl, der von der Grundbaugruppe abgestrahlt wird, unmittelbar von der Laserdiode abgestrahlt wird und sich mit einem ersten Divergenzwinkel und mit einem zweiten Divergenzwinkel, der größer ist als der erste Divergenzwinkel, ausbreitet und
die Kollimatorlinse eine längliche torische Linse ist, die eine größere Ausdehnung aufweist, die mit dem zweiten Divergenzwinkel ausgerichtet ist.

## Revendications

1. Appareil laser thérapeutique, comprenant :
une enveloppe (11) adaptée pour être tenue à la main ;
un assemblage de base (21, 31) dans l'enveloppe, l'assemblage de base comprenant une diode laser (20, 30, 40, 61) configurée pour générer un faisceau laser monochromatique, l'assemblage de base étant configuré pour émettre un faisceau laser divergent ; et
au moins deux assemblages de tête pouvant s'engager de manière amovible dans l'assemblage de base, chaque assemblage de tête comprenant :
un support de lentille (22, 38) ayant une dimension axiale (D) ; et
une lentille de collimation (23, 37, 64) sur le support de lentille, configurée pour générer un faisceau collimaté final approximativement cohérent ;
la dimension axiale du support de lentille d'un premier des assemblages de tête étant différente de la dimension axiale du support de lentille d'un deuxième des assemblages de tête, et une dimension de la lentille de collimation du premier des assemblages de tête étant différente d'une dimension de la lentille de collimation du deuxième des assemblages de tête ;
lors de l'engagement du premier des au moins deux assemblages de tête dans l'assemblage de base, la lentille de collimation du premier assemblage de tête intercepte, à une première distance de l'assemblage de base, le faisceau divergent émis par l'assemblage de base, et lors de l'engagement du deuxième des au moins deux assemblages de tête dans l'assemblage de base, la lentille de collimation du deuxième assemblage de base intercepte, à une deuxième distance de l'assemblage de base, différente de la première distance, le faisceau divergent émis par l'assemblage de base ;
lors de l'utilisation avec l'assemblage de base, le premier assemblage de tête crée un premier faisceau collimaté final ayant une dimension de faisceau latérale différente d'une dimension de faisceau latérale correspondante d'un deuxième faisceau collimaté final créé par le deuxième assemblage de tête lors de l'utilisation avec l'assemblage de base.

2. Appareil selon la revendication 1, dans lequel ;
les au moins deux assemblages de tête comprennent plusieurs assemblages de tête interchangeables par rapport à l'assemblage de base ; et
un quelconque des assemblages de base, lors de l'utilisation avec l'assemblage de base, émet un faisceau collimaté final, sensiblement cohérent, ayant un flux de rayonnement différent d'un flux de rayonnement émis par un quelconque autre des assemblages de tête lors de l'utilisation avec l'assemblage de base.

3. Appareil selon la revendication 1, dans lequel :
le faisceau laser divergent émis par l'assemblage de base est émis directement par la diode laser, et se propage à un premier angle de divergence et à un deuxième angle de divergence supérieur au premier angle de divergence ; et
la lentille de collimation est un lentille toroïdale oblongue ayant une dimension majeure alignée avec le deuxième angle de divergence.

4. Appareil selon la revendication 1, dans lequel le faisceau final forme, sur un plan perpendiculaire à la direction de propagation du faisceau, une zone éclairée allongée, la longueur de la zone éclairée représentant au moins deux fois la largeur de la zone éclairée.

5. Appareil selon la revendication 1, dans lequel l'assemblage de base comprend en outre :
une lentille de correction (32, 62) configurée pour corriger l'astigmatisme dans un faisceau divergent d'origine émis par la diode laser et pour modifier le faisceau d'origine en vue de créer un faisceau cohérent approximativement circulaire ; et
une lentille divergente (33, 63) configurée pour créer, à partir du faisceau cohérent créé par la lentille de correction, le faisceau laser divergent émis par l'assemblage de base ;
le faisceau laser divergent émis par l'assemblage de base étant un faisceau divergent approximativement circulaire.

6. Appareil selon la revendication 5, dans lequel la lentille de collimation sur le support de lentille émet un faisceau final approximativement circulaire.

7. Appareil selon la revendication 1, comportant une ouverture laser active ajustable, l'assemblage de base comprenant en outre :
une lentille de correction (32, 62) configurée pour modifier un faisceau divergent d'origine émis par la diode laser pour corriger un astigmatisme de faisceau et créer un faisceau collimaté cohérent approximativement circulaire ; et
une lentille divergente (33, 63) configurée pour créer, à partir du faisceau cohérent créé par la lentille de correction, le faisceau laser divergent émis par l'assemblage de base, le faisceau laser divergent émis par l'assemblage de base étant approximativement circulaire ;
les assemblages de tête étant interchangeables par rapport à l'assemblage de base ;
lors de l'engagement du premier des au moins deux assemblages de tête dans l'assemblage de base, la lentille de collimation du prem1ier assemblage de tête intercepte, à la première distance de l'assemblage de base, sensiblement l'ensemble du faisceau divergent émis par l'assemblage de base, et lors de l'engagement du deuxième des au moins deux assemblages de tête dans l'assemblage de base, la lentille de collimation du deuxième assemblage de tête intercepte, à la deuxième distance de l'assemblage de base, différente de la première distance, sensiblement l'ensemble du faisceau divergent émis par l'assemblage de base ;
lors de l'utilisation avec l'assemblage de base, un quelconque des assemblages de tête émet un faisceau final collimaté sensiblement cohérent, ayant un flux de rayonnement différent d'un flux de rayonnement émis par un quelconque autre des assemblages de tête, lors de l'utilisation avec l'assemblage de base.

8. Appareil selon la revendication 1,
dans lequel la lentille de collimation sur le support de lentille est configurée pour créer un faisceau final collimaté sensiblement cohérent, le faisceau final définissant, sur un plan perpendiculaire à la direction de propagation du faisceau, une zone éclairée allongée, la longueur de la zone éclairée représentant au moins deux fois la largeur de la zone éclairée.

9. Appareil selon la revendication 8, dans lequel :
les au moins deux assemblages de tête comprennent plusieurs assemblages de tête interchangeables par rapport à l'assemblage de base ; et
un quelconque des assemblages de tête est configuré, lors de l'utilisation avec l'assemblage de base, pour émettre un faisceau final sensiblement cohérent ayant un flux de rayonnement différent d'un flux de rayonnement émis par un quelconque autre des assemblages de tête, lors de l'utilisation avec l'assemblage de base.

10. Appareil selon la revendication 8, dans lequel :
le faisceau laser divergent émis par l'assemblage de base est émis directement par la diode laser, et se propage à un premier angle de divergence et à un deuxième angle de divergence supérieur au premier angle de divergence ; et
la lentille de collimation est une lentille toroïdale oblongue ayant une dimension majeure alignée avec le deuxième angle de divergence.
